# EUROPEAN PATENT APPLICATION

(11) **EP 2 098 166 A1**
(43) Date of publication of application: **09.09.2009**
(21) Application number: 09153979.1
(22) Date of filing: 27.02.2009
(51) Int. Cl.: A61B 5/097, A61B 5/087

(54) **Mouthpiece**

(30) Priority: 29.02.2008 GB 0803749
(71) Applicant: Bedfont Scientific Limited, Rochester, Kent ME1 3QX (GB); Lascar Electronics, Salisbury, Wiltshire SP5 2SJ (GB)
(72) Inventor: Smith, Trevor, Maidstone, Kent ME17 3EZ (GB); Bromley, Phillip, Rainham, Kent ME8 8EP (GB); Cross, Pete, Alderbury, Wiltshire SP5 3PA (GB)
(74) Representative: Jones, Keith William

(57) **Abstract**

A mouthpiece capable of being compressed to allow comparatively low volume packing, the mouthpiece having a tube comprising a wall defining a lumen, and the wall comprising a region of resilient material which allows the tube to collapse when sufficient pressure is applied to the wall the of tube. Such a mouthpiece may also be suitable for use in cooperation with an apparatus used for analysing exhaled breath.

## Description

The present invention relates to a mouthpiece, in particular to a mouthpiece which is capable of being compressed to allow comparatively low volume packing. The invention further relates to a combination of a mouthpiece in cooperation with an apparatus used for analysing the exhaled breath of a user.

Apparatuses for analysing the exhaled breath of a user are commonly used in medical and other fields. Spirometers, peak flow meters, and gas analysis devices are examples of such apparatuses.

When using such devices hygiene and infection control is a significant issue, particularly where a number of users are to use the same device. In order to ensure hygiene between users it is good practice to use a replaceable mouthpiece which can be changed between users. These mouthpieces can be disposable.

Such replaceable mouthpieces are typically formed from plastic or cardboard, and may be packaged individually. Because of the generally tubular form of the mouthpieces, when packaged the product takes up a large volume, most of which is air. This is highly inefficient and expensive in terms of both transportation and storage.

According to a first aspect the present invention provides a mouthpiece comprising a tube having a wall defining a lumen, the wall comprising a region of resilient material which allows the tube to collapse when sufficient pressure is applied to the wall of the tube.

Suitable resilient materials include plastics, cardboard or paper.

In one embodiment the tube is formed entirely from a resilient material. The tube can be substantially formed from a single material, but with regions of reduced thickness or other structural modifications (e.g. serrations, scoring etc.) to allow collapse to be easily achieved via these regions. Alternatively, regions of the tube could be formed from a comparatively rigid material, with regions of the tube being formed from resilient material to allow the tube to be collapsed; such resilient regions may suitably comprise two or more longitudinal regions extending along the length of the tube, e.g. two diametrically opposed resilient regions.

In a preferred embodiment the tube is formed substantially entirely from a single resilient material, optionally with longitudinal regions of reduced thickness or rigidity.

The tube may conveniently be formed from a sheet of material which is rolled to form a tube, and then secured in this tubular form. The sheet of material may conveniently be secured in a tubular configuration by applying adhesive to a region of the sheet which overlaps a region of the opposite edge of the sheet when rolled into the tube. Suitable adhesives for such a purpose are well known in the art.

In a preferred embodiment of the present invention the tube is formed from paper or cardboard, which is glued into a tubular configuration. Suitably longitudinal scores are provided in the paper or cardboard to facilitate collapse of the tube along desired longitudinal regions.

In one preferred embodiment the tube is formed a sheet material, which is coiled in a helical configuration and secured. The tube formed from such a helical configuration may be likened to that of domestic toilet roll support tube.

In a preferred embodiment, the mouthpiece can be formed through compressing a thinly walled tube with no joins to form a flat mouthpiece.

Suitably, the mouthpiece is formed from a tube produced through rolling material into a tube and then cutting the tube thus formed into a suitable length. Preferably, this material is cardboard, between 0.5 to 1.5 mm thick, but can be another material such as plastic.

It is generally preferred that the mouthpiece comprises an anti-microbial material to prevent or reduce the proliferation of microbes on and/or in the mouthpiece. For example the tube might be coated, impregnated, or substantially entirely formed from an anti-microbial material. Such materials are well known in the art and include, for example, silver or silver salts, and antimicrobial polymers. A particularly preferred material is a paper or cardboard treated with an antimicrobial substance.

Where paper or cardboard is used, it has been found that a weight of from around 200 to 400 g/m² is generally suitable, preferably around 250 to 350 g/m², especially around 300 g/m². Such a material has beneficial properties in that it is easy to work and handle, cheap to produce, can be formed from recycled material, and has a long shelf life. The cardboard is preferred to be a food grade material.

In a preferred embodiment the mouthpiece of the present invention is relatively easy to collapse, such that in order to be used as a mouthpiece it requires structural support from the apparatus to which it is attached. In other words, the mouthpiece does not need to be inherently resilient enough that it is urged into the tubular conformation with sufficient force that it can itself resist collapse under the pressure of a users lips pressing upon it when in use. The necessary strength to allow the mouthpiece to function (i.e. remain in an open confirmation) can be, at least in part, derived from its interface with the apparatus of breath testing equipment.

Suitably the length of the tube of the mouthpiece is from around 30 mm to around 90 mm, preferably from around 40 mm to around 75 mm, especially around 50 mm.

The tube may be of any suitable cross-section, although generally round or elliptical cross-sections are preferred. The cross-section may vary along the length of the tube, e.g. from circular to elliptical, but this is not usually an advantage. Tubes of elliptical cross-section at the end that the user contacts have been found to be advantageous as they are well adapted to allow a comfortable and airtight seal to be achieved by the lips of the user.

The dimensions of the mouthpiece should be chosen such that it is suitable for use by a user, i.e. it should be sized such that a user can comfortably fit the tube into their mouth and form a seal around the tube with their lips. A diameter of from around 0.75 to inches around 1.5 inches (approx. 19mm to 38 mm) in a tube of circular cross-section, or a similar major axis in a tube of elliptical cross-section, has been found to be a suitable size, but other sizes outside of this range may also be suitable.

In one embodiment the wall of the tube comprises a laminate material, which contains two layers with a middle layer sandwiched therebetween. The middle layer may suitably comprise information carrying means. The purpose of the information carrying means is to allow details regarding the product to be provided within the structure of the mouthpiece in such a way that is tamper proof and difficult to copy. Suitable information might include the manufacturer, date of manufacture, serial or other identifying code, certification of authenticity etc.

In a further aspect the present invention provides one or more mouthpieces as described above packaged within a hermetically sealed package. Preferably the mouthpiece(s) are sterilised, and the package is capable of maintaining the sterility of the mouthpiece.

Suitably the package is formed from a plastics material (e.g. polypropylene) or a coated paper material.

The mouthpiece(s) may be packaged individually, i.e. one per hermetically sealed package, or two or more may be provided in a single package.

It is an advantage that, when packaged, the mouthpieces are in a flat packed form, thus allowing for very space efficient packaging of the mouthpieces for transportation and storage. Prior art mouthpieces made of rigid material are permanently in an open configuration, which means that packaging in a space efficient manner is impossible. This results in high transportation and storage costs. In addition, the efficient packaging of the flat-packed mouthpieces, over conventional tubular mouthpieces, will produce a lower environmental cost. This is due to the smaller packaging required to contain the mouthpieces, and the reduced space for shipping.

In a further aspect the present invention provides a mouthpiece as described above in cooperation with an apparatus used for analysing the exhaled breath of a user. Examples of such apparatuses include, for example, spirometers, peak flow meters, breath gas monitors, including carbon monoxide (CO) and hydrogen (H₂), and NO level analysers.

In a preferred embodiment the apparatus comprises an adaptor to interface with the mouthpiece. Suitably the adaptor is shaped and sized such that it will interface with the mouthpiece tightly, so that there is, at most, a negligible escape of exhaled air between the adaptor and the mouthpiece when a user exhales through the mouthpiece into the apparatus.

Suitable cross-sections for the adaptor include circular or elliptical.

The adaptor may suitably be provided by an annular (e.g. circular or elliptical ring) ridge extending from the apparatus. It is preferred that when the mouthpiece is connected to the adaptor, it slides over the outside of the adaptor, i.e. the adaptor forms a male part of the interface and the mouthpiece the female part. Where such an arrangement is provided the adaptor serves to support the structure of the mouthpiece. It is possible to connect the mouthpiece such that it slides inside the adaptor, but this arrangement is less supportive to the mouthpiece, and thus requires the use of a more robust material for the mouthpiece. A third arrangement is that the adaptor comprise two annular (e.g. circular or elliptical) ridges, which are substantially concentric, the ridges defining an annular groove into which the mouthpiece can be mounted; this arrangement also provides a high level of support for the mouthpiece, but is potentially more complex to manufacture. It will be clear to the person skilled in the art that the respective sizes of the mouthpiece and the adaptor should be chosen such that a relatively tight, and hence suitable air-tight seal between the mouthpiece and the adaptor is achieved.

Optionally the adaptor may be tapered such that the fit between the mouthpiece and the adaptor becomes tighter the further onto the adaptor the mouthpiece is slid.

In a further embodiment the adaptor or apparatus is provided with one or more projections or depressions which are adapted and positioned such that they will come into contact with the mouthpiece when it is mounted in position. The mouthpiece is provided with corresponding projections or depressions such that it interacts with the adaptor in a lock and key fashion. This feature can have a number of functions, for example, 1) it can be used to ensure that only the correct mouthpieces are attached to the apparatus, as incorrect mouthpieces will not fit properly, even if their dimensions are similar; and 2) it can be used to ensure the mouthpiece is connected in a proper orientation.

An embodiment of the present invention will now be described, by way of example only, with reference to the accompanying drawings, in which:

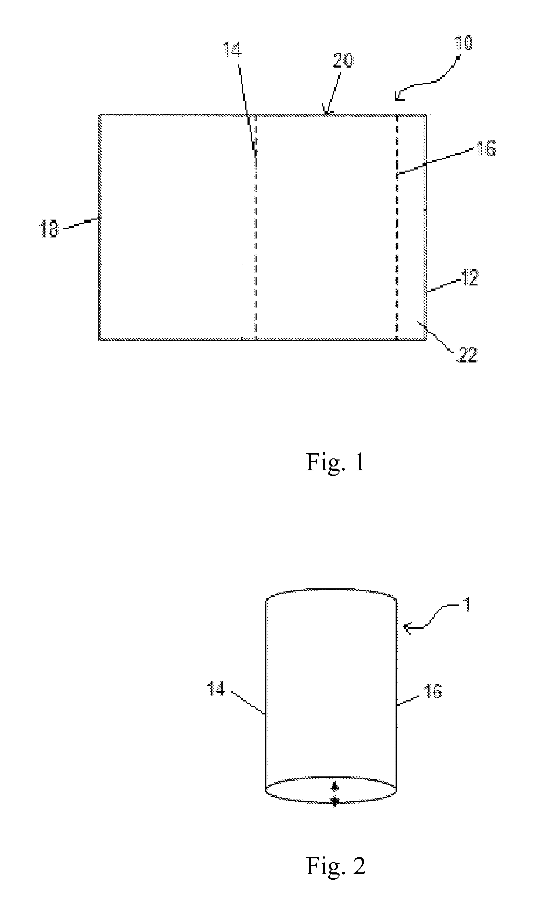

- Fig. 1 shows a net of the mouthpiece, i.e. a rectangular structure which is rolled to form the tubular mouthpiece; and
- Fig. 2 shows a mouthpiece according to the present invention.

A net 10 for the construction of a mouthpiece 1 according to the present invention comprises a rectangular section 12 of paper of 300 g/m² weight, of length 50 mm and width 79 mm. Two longitudinal scores 14,16 are provided on the rectangle 12, the first score 14 provided 34.5 mm from a first short edge 18 of the rectangle 12, the second 16 a further 34.5 mm from the first edge 18. Thus the rectangle 12 is effectively split by the longitudinal scores 14,16 into two adjacent sections of 50 mm by 34.5 mm, with a third section of 11 mm being defined by the second 16 score and the second short edge of the rectangle 20. The third section provides a flap 22 which is used to secure the mouthpiece 1 in a tubular configuration.

The net 10 is formed into a tube by rolling it along its length to form a tube of length 50 mm. The flap 22 is secured with glue to the first edge 18 of the tube to hold the mouthpiece 1 in a tubular form.

The scores 14,16 provide points of increased flexibility which allow simple collapsing of the mouthpiece 1 into a flat conformation for packaging. To collapse the mouthpiece 1, pressure is applied to the tube, preferably at a central point between the scores. The mouthpiece 1 is then in a suitable form for packaging in conventional techniques used for packaging medical items. The mouthpiece 1 may be sterilised before, during or after the packaging step.

In use the mouthpiece 1 is removed from the packaging. To encourage the mouthpiece 1 to regain an open tubular conformation, pressure can be applied to the edges of the mouthpiece 1. This pressure will cause the mouthpiece 1 piece to open.

The mouthpiece 1 may then be slid onto an adaptor on an apparatus used for analysing the exhaled breath of a user. A suitable example of such an apparatus is a breath analyser for a gas such as CO or H₂. The apparatus is provided with an adaptor which is an elliptical collar of slightly smaller overall dimensions than the mouthpiece, but large enough that a tight fit between the mouthpiece 1 and the adaptor is formed when the mouthpiece 1 is slid thereupon. For use with the mouthpiece 1 of the described above an elliptical adaptor of 24.15 mm by 18.52 mm (in the major and minor axes respectively) is suitable. The adaptor may be tapered to ensure an air-tight fit is achieved.

The user can then blow through the mouthpiece 1 into the apparatus as required. Following use the mouthpiece can be discarded and another mouthpiece 1 used for the next user.

## Claims

1. A mouthpiece comprising a tube having a wall defining a lumen, the wall comprising a region of resilient material which allows the tube to collapse when sufficient pressure is applied to the wall of a tube.

2. A mouthpiece according to claim 1, wherein said resilient material comprises plastic, cardboard or paper.

3. A mouthpiece according to claim 1 or 2, wherein said tube is formed substantially entirely from a single resilient material.

4. A mouthpiece according to any proceeding claim, wherein said tube is provided with longitudinal regions of reduced thickness or rigidity.

5. A mouthpiece according to any proceeding claim, wherein said mouthpiece comprises anti-microbial material.

6. A mouthpiece according to any one of claims 2 to 5, wherein said resilient material is paper or cardboard having a weight of from around 200 to 400 g/m².

7. A mouthpiece according to any proceeding claim, wherein said tube has a length from around 30 mm to 90 mm.

8. A mouthpiece according to any proceeding claim, wherein said tube wall comprises a laminate material.

9. A mouthpiece according to claim 8, wherein said laminate material contains two layers with a middle layer sandwiched therebetween, said middle layer comprising an information carrying means.

10. A mouthpiece according to any one of claims 1 to 9 packaged within a hermetically sealed package.

11. A mouthpiece according to any one of claims 1 to 9, in cooperation with an apparatus used for analysing the exhaled breath of a user.

12. A mouthpiece according to claim 11, wherein said apparatus comprises an adaptor to interface with the mouthpiece.

13. A mouthpiece according to claim 12, wherein said adaptor is provided by an annular ridge extending from the apparatus.

14. A mouthpiece according to claim 12, wherein said adaptor comprises two annular ridges defining an annular groove.

15. A mouthpiece according to any one of claims 11 to 14, wherein said apparatus or adaptor is provided with one or more projections or depressions adapted and positioned such that they will come into contact with a mouthpiece when it is mounted in position; and wherein said mouthpiece is provided with corresponding projections or depressions such that it interacts with the adaptor or apparatus in a lock and key fashion.
